# EUROPEAN PATENT APPLICATION

(11) **EP 1 733 710 A1**
(43) Date of publication of application: **20.12.2006**
(21) Application number: 05013123.4
(22) Date of filing: 17.06.2005
(51) Int. Cl.: A61K 8/04

(54) **Cohesive emulsion composition comprising a bleaching agent**

(71) Applicant: THE PROCTER & GAMBLE COMPANY, Cincinnati, Ohio 45202 (US)
(72) Inventor: Romano, Mario, 65125 Pescara (IT); James, Martin., 65016 Montesilvano Colle (Pescara) (IT)
(74) Representative: Veronese, Pancrazio

(57) **Abstract**

The present invention relates to a cohesive emulsion comprising a bleaching agent, an emulsifying system, an aqueous phase and an inert hydrophobic phase, wherein the hydrophobic (e.g. oil) phase is in predominant proportion relative to the aqueous phase. In one embodiment the invention relates to a delivery system for delivering a bleaching agent to the teeth surface comprising the present compositions and an integral carrier, e.g. a strip of material. In another embodiment the present invention relates to a method of preparation of emulsions.

## Description

The present invention relates to a cohesive emulsion comprising a bleaching agent, an emulsifying system, an aqueous phase and an inert hydrophobic phase, wherein the hydrophobic (e.g. oil) phase is in predominant proportion relative to the aqueous phase. In one embodiment the invention relates to a delivery system for delivering a bleaching agent to the teeth surface comprising the present compositions and an integral carrier, e.g. a strip of material.

### BACKGROUND OF THE INVENTION

Dental products by which various cosmetic and/or therapeutic actives are delivered to teeth and the oral cavity are known. Examples of such products include: brushing aids such as dentifrice products for delivery of oral care actives such as polyphosphates or fluorides; mouthwashes containing breath fresheners or antibacterial actives; and whitening strips for the delivery of bleaching actives to the teeth. In particular the use of a dental strip has been recognized as a convenient and inexpensive way to deliver cosmetic and therapeutic benefits to the teeth and mucosal surfaces of the oral cavity. For example, dental whitening strips, where a whitening composition is applied to a strip and thereafter applied to the teeth to achieve sustained contact between the teeth and the whitening composition, are known. See U.S. Pat. Nos. 6,136,297; 6,096,328; 5,894,017; 5,891,453; and 5,879,691, all to Sagel, et al., and U.S. Pat. Nos. 5,989,569 and 6,045,811 both to Dirksing, et al., all assigned to The Procter & Gamble Company.

Despite the above known approaches for the treatment of oral conditions, especially for the whitening of teeth, a need still exists for providing products with both improved bleaching efficacy as well as increased speed of whitening. The prior art has generally attempted to address these concerns by increasing the level of the bleaching agent in the compositions. This approach, however, presents several problems. First the subject may experience increased irritation and/or sensitivity which may be associated with using an increased amount of a bleaching agent. Furthermore, some regulatory authorities and legislation in various geographies throughout the world do not allow bleaching agents to be used in products at levels above certain concentrations. Therefore, despite the above known approaches for the treatment of oral conditions, especially for the whitening of teeth, a need still exists for providing products with improved bleaching efficacy.

A novel approach to this problem which overcomes some of the limitations of the prior art, is described in our co-pending application number PCT/US2004/042973 entitled "Emulsion Composition for Delivery of Bleaching Agents to Teeth" which relates to a composition comprising a bleaching agent, an emulsifying system, an aqueous phase and an inert hydrophobic phase, wherein the inert hydrophobic phase is in predominant proportion relative to the aqueous phase.

The compositions described in the mentioned application are in general pasty or creamy and therefore are not usable in those situations where a cohesive material is desirable.

The compositions of the present invention not only provide improved bleaching efficacy but also are provided in the form of a semi solid cohesive gel. Such compositions are particularly useful e.g. for use on teeth bleaching strips, cohesive material in fact do not require a carrier material such as for example a sponge onto which it can be absorbed, thus allowing a much simpler manufacturing process and a simpler product, where the composition is directly applied onto the strip.

### SUMMARY OF THE INVENTION

The present invention relates to a cohesive emulsion useful whitening teeth comprising:
a. An aqueous phase in an amount from 1% to 45%, preferably from 3% to 35% by weight of the composition
b. A safe and effective amount of a bleaching agent;
c. an inert hydrophobic phase in an amount from 30% to 98%, preferably from 40% to 90% by weight of the composition
d. an emulsifying system in an amount from 1% to 10%, preferably from 3% to 7% by weight of the composition,
said composition being characterized in that said emulsifying system comprises at least one surfactant having an HLB<10, preferably comprised between 3 and 7, and at least one surfactant with HLB>10, preferably comprised between 12 and 16.

In another embodiment the present invention also relates to an oral care delivery system comprising:
a. an integral carrier; and
b. a safe and effective amount of an emulsion as described above.

In one embodiment the delivery system comprises: a first layer of a strip of material; a second layer comprising the present composition, whereby the bleaching agent is releasably associated with the strip of material. The present invention can be used to deliver whitening benefits to the oral cavity by directly applying the composition or the integral carrier to the teeth. The integral carrier is attached to the teeth via the compositions herein or the adhesion function can be provided independent of the present compositions herein (e.g. can be provided via a separate adhesive composition used with the present compositions and integral carrier or via addition of a tackifier resin to the emulsion composition). Alternatively, the integral carrier may be attached to the teeth via an attachment means that is part of the integral carrier, for example the integral carrier may optionally be of sufficient size that, once applied, the integral carrier overlaps with the oral soft tissues rendering more of the teeth surface available for bleaching.

### DETAILED DESCRIPTION OF THE INVENTION

### Definitions

By "oral care composition" or "oral composition" as used herein is meant a product which is not intentionally swallowed for purposes of systemic administration of therapeutic agents, but is retained in the oral cavity for a sufficient time to contact the dental surfaces for purposes of whitening efficacy.

By "safe and effective amount" as used herein is meant an amount of a component, high enough to significantly (positively) modify the condition to be treated or to effect the desired whitening result, but low enough to avoid serious side effects (at a reasonable benefit/risk ratio), within the scope of sound medical/dental judgment. The safe and effective amount of a component, will vary with the particular condition (e.g., to effect whitening) being treated, the age and physical condition of the patient being treated, the severity of the condition, the duration of treatment, the nature of concurrent therapy, the specific form employed, and the particular vehicle from which the component is applied.

By "a sufficient period of time to achieve whitening" as used herein is meant that the composition is used or worn by the subject or the subject is instructed to use or wear the composition for greater than about 2 minutes, in another embodiment from about 2.5 minutes to about 12 hours (e.g overnight treatment), in another embodiment from about 3 minutes to about 120 minutes, in yet another embodiment from about 5 minutes to about 40 minutes, per application, and may be applied from about 1 to about 7 times per day. Additionally, the length of treatment to achieve the desired benefit, for example, tooth whitening, may last from about one day to about six months, in another embodiment from about one day to about 28 days, and in another embodiment from about 7 to about 28 days. The optimal duration and frequency of application will depend on the desired effect, the severity of any condition being treated, the health and age of the user and like considerations.

All percentages and ratios used herein after are by weight of total composition, unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

### Emulsion Compositions

Emulsions as those described in our co-pending application number PCT/US2004/042973 are generally of the type "oil in water" or "water in oil". In the case of oil in water emulsions aqueous phase is the continuous phase and the oil phase is dispersed in small droplets. The aqueous phase is preferred to be in general a low viscosity phase so that the diffusion of the bleaching agent to the teeth surface is not impaired. Therefore these types of emulsion are in general liquid and non cohesive.

"Water in oil" emulsions are constituted by a continuous inert hydrophobic phase wherein small aqueous phase droplets are dispersed. The continuous hydrophobic phase can be formulated so that it provides a solid or semi solid framework for the emulsion, however, in the case of a water in oil emulsion, the droplets of aqueous phase which contain the bleaching agent must be relatively free to move in order to reach the target surface while said solid or semi-solid framework provided by the hydrophobic phase substantially reduces the movement of the droplets of aqueous phase, thus substantially reducing also the bleaching efficacy of the composition.

Surprisingly, compositions formulated according to the teaching of the present invention are semi-solid and cohesive, but at the same time provide a similar bleaching efficacy if compared to the less cohesive compositions described in our co-pending application number PCT/US2004/042973.

Without being bound to theory it is believed that the emulsions of the present invention have a bicontinuous structure wherein the two phases are completely interpenetrated. In a bicontinuous structure , similarly to a sponge, the oil phase can provide the cohesive framework in which the non cohesive aqueous phase is free to move and to reach the teeth surface.

The compositions according to the present invention comprise an aqueous phase, an immiscible inert hydrophobic phase, a bleaching agent, as well as an emulsifying system.

The inert hydrophobic phase may provide a more stable matrix for flavor ingredients, especially those flavor ingredients that are soluble in the inert hydrophobic phase.

Lastly, the emulsion may also form a "film" over the soft tissues of the oral cavity such as the gums, to decrease tooth sensitivity that is sometimes associated with bleaching agents applied in the oral cavity.

### Aqueous Phase

The present compositions comprise a safe and effective amount of an aqueous phase comprising water and/or water dispersible/miscible liquids. The level of the aqueous phase, is 1 % to 45%, preferably from 3% to 35%, more preferably from 10% to 25% by weight of the composition. In another embodiment very low levels of aqueous phase may be useful, for example, from 1% to 10%, in another embodiment from 2% to 8%, in another embodiment from 2% to 5%, by weight of the composition.

The aqueous phase preferably comprises water, polyalkylene glycols with molecular weights from about 200 to about 20,000, humectants, and mixtures thereof. Humectants generally include edible polyhydric alcohols such as glycerin, sorbitol, xylitol, butylene glycol, polyethylene glycol, and propylene glycol, and mixtures thereof. Preferably the aqueous phase comprises more than 20% by weight of the aqueous phase of water, more preferably more than 40% by weight of the aqueous phase of water. In one embodiment the aqueous phase comprises at least about 10% of water, in another embodiment at least about 20% by weight of the aqueous phase, of water.

### Bleaching Agent

The present compositions further comprise a safe and effective amount of a bleaching agent. In one embodiment the level of bleaching agent is dependent on the available oxygen or chlorine respectively that the molecule is capable of providing to bleach the stain. The bleaching agent level is preferably from 0.1 % to 20%, more preferably from 0.5 to 9% and most preferably from 3% to 8% by weight of the composition, of the bleaching agent. In one embodiment the bleaching agent is surprisingly more effective when used at lower levels, generally from 0.5% to 3%, in another embodiment from 0.5% to 1.5% by weight of the composition.

In one embodiment the bleaching agents are selected from the group consisting of the peroxides, metal chlorites, perborates, percarbonates, peroxyacids, persulfates, compounds that form the preceding compounds in situ, and combinations thereof. Suitable peroxide compounds include hydrogen peroxide, urea peroxide, calcium peroxide, carbamide peroxide, and mixtures thereof. Preferably the bleaching agent is hydrogen peroxide.

### Inert Hydrophobic Phase

The present compositions comprise a safe and effective amount of an inert hydrophobic phase. The inert hydrophobic phase is a hydrophobic material, which is water insoluble or water immiscible, non-toxic to the user and chemically stable and compatible with other ingredients present in the composition. The present composition comprises from 30% to 98%, preferably from 40% to 90% and more preferably from 50% to 85% and even more preferably from 65% to 80% by weight of the composition, of the inert hydrophobic phase as the continuous phase. In one embodiment the inert hydrophobic phase is in predominant proportion relative to the aqueous phase present in the composition. As used herein "predominant proportion" means that the percent by weight of the composition of the inert hydrophobic phase is in excess relative to the percent by weight of the composition of the aqueous phase

The hydrophobic phase preferably includes a polymeric component and a hydrophobic oil. The polymeric component is preferably a block or graft copolymer, most preferably having at least one polyolefin segment compatible with the oil and one segment incompatible with the oil. Preferred polymeric components for use in the present invention comprise styrenic block copolymers, preferably wherein the soft segment of said styrenic block copolymers is a saturated olefin rubber, more preferably wherein said saturated olefine rubber is selected from a a polyethylene/polybutylene rubber, polyethylene/polypropylene rubber and mixtures thereof. Most preferred styrenic block copolymers are those marketed under the tradename of Kraton®.

The hydrophobic oil component is preferably selected to solubilise one segment of said polymeric component. Suitable oils to be comprised in said hydrophobic oil component include mineral oil, castor oil, linseed oil, rape oil, peanut oil, soybean oil, rice bran oil, coconut oil, palm oil, safflower oil, olive oil, vegetable oils, corn oil, sesame oil, hydrogenated castor oil, partially hydrogenated soybean oil, glyceryl trioleate, glyceryl trilinoleate, a Ω3 polyunsaturated fatty acid triglyceride containing oil, and mixtures thereof. Preferably the hydrophobic oil is a mineral oil.

### Emulsifying system

The present compositions comprise an amount from 1% to 10%, preferably from 3% to 7% by weight of an emulsifying system.

According to the present invention the emulsifying system comprises at least one surfactant having an HLB<10, preferably comprised between 3 and 7, and at least one surfactant with HLB>10, preferably comprised between 12 and 16.

Any surfactant having HLB ratios as described above may be used as long as is non-toxic to the user, chemically stable and compatible with other ingredients present in the composition, especially the bleach.

Preferred surfactants are non reactive with the bleach agent. For example, surfactants that are non-reactive with the bleach agent, are free of nitrogen groups and linkages, are essentially free of metals such as Fe, Zn, etc.

Preferred surfactants having an HLB<10 are non ionic surfactants, preferably fatty acid esters or sugars, most preferred surfactant is sorbitan monooleate. Said surfactants are preferably present in an amount from 0.9% to 9.9%, preferably from 3% to 7%, by weight of the total composition.

Preferred surfactants having an HLB>10 are polymeric non ionic surfactants, preferably block copolymers of polyoxyethylene and polyoxypropylene, most preferably having a molecular weight from 2000 to 5000 and a polyoxyethylene content from 40% to 60%. Said surfactants are preferably present in an amount from 0.1 % to 5%, preferably from 0.3% to 3%, by weight of the total composition.

### Viscosity of the Emulsion Composition

The composition of the present invention may be in the form of a visco-elastic semi-solid or gel. Preferably the composition has a complex viscosity of from about 200 to about 1,000,000 cps at low frequency (10 rad/s), more preferably from about 100,000 to about 800,000 cps and even more preferably from about 400,000 to about 600,000 cps. Viscosities are measured using standard oscillatory rheometric techniques at room temperature which are known to those skilled in the art. The viscosities of the final products were measured at 25 degrees C using a Rheometrics rheometer (model SR5000) using a 25mm parallel plate geometry at a constant frequency of 10 rad/s and a stress selected such that the sample is within the linear viscoelastic region.

### Optional components

The Compositions of the present invention may further optionally comprise thickening agents, tackifier resins, colorants, perfumes, flavoring agents oral care active agents (anticalculus agents, H2 antagonists, anti-inflammatory agents, antimicrobial agents, desensitizing agents, nutrients) and the like, as described in our co-pending application number PCT/US2004/042973.

### Combination of Integral Carrier and Emulsion Composition

In one embodiment the present invention relates to a delivery system comprising the present compositions used with an integral carrier. Typically an integral carrier comprises a strip of material, e.g. a plastic film, onto which the composition of the present invention is provided. Preferably the delivery system comprises: a first layer of a strip of material; a second layer comprising the present composition described herein, whereby the bleaching agent is releasably associated with the present composition. All combinations of emulsions and integral carrier described in our co-pending application number PCT/US2004/042973 can be used in the present invention. The compositions of the present invention are particularly suitable in those embodiments wherein said second layer of composition is directly applied on a substrate layer such as for example a plastic film.

Flavorants or sweeteners may also be added to the second layer composition by mixing as desired. Thereafter the composition is added to the integral carrier, as desired.
The integral carrier, such as a strip, may be formed by several of the film making processes known in the art. In one embodiment a strip of polyethylene is made by a blown process or a cast process. Other processes including extrusion or processes that do not affect the flexural rigidity of the strip of material are also feasible. Additionally, the second layer composition may be incorporated onto the strip during the processing of the strip. The second layer composition may be a laminate on the strip.

### Methods of Manufacturing Emulsion Compositions

Due to the highly viscoelastic nature of the hydrophobic phase of the compositions of the present invention, common emulsification methods are not effective in preparation of the current formulations.

In one embodiment the present invention encompasses a method of making an emulsion which comprises the steps of:
a) combining an oil gel with a surfactant having an HLB<10 to form an hydrophobic phase.
b) combining water, a bleaching agent and a surfactant having an HLB>10 to form an aqueous phase
c) combining said hydrophobic phase and said aqueous phase in a twin screw extruder to form an emulsion.

Step a) is preferably performed at a temperature ranging from 50°C to 100°C, step c) is preferably performed at a temperature ranging from 10°C to 60°C, more preferably from 20°C to 40°C, most preferably from 30°C to 40°C. Preferably all mixing equipments do not introduce metal ions contamination which may lead to bleach degradation. Materials that can be used include e.g. glass, plastic, Teflon coated materials and stainless steel

### Methods of Using the Compositions and/or Delivery Systems

The present invention can be applied to the teeth of a consumer in the dental office by a dental professional, or can be used at home by the consumer. Generally, the recommended treatment period is, in one embodiment, a sufficient period of time to achieve whitening.

In practicing the present invention, the user applies the composition herein that contains the bleach to obtain the desired effect, e.g., whitening, to one or more teeth. The composition is preferably combined with an integral carrier such as a strip of material, or a dental tray, and thereafter applied to the teeth.

It is not necessary to prepare the teeth before applying the present invention. For example, the user may or may not choose to brush the teeth or rinse the mouth before applying the present invention. The surfaces of the oral cavity are neither required to be dried nor to be excessively wet with saliva or water before the application. However, it is believed that adhesion to the tooth enamel surfaces will be improved if the teeth are dry prior to application.

Where the integral carrier is a strip of material, the second layer composition may be coated on the strip of material, or be applied by the user to the strip of material, or be applied by the user to the teeth and then the strip of material placed over the coated teeth. The amount of the second layer composition applied to the strip of material or teeth may depend upon the size and capacity of the strip of material, concentration of the active and the desired benefit. Generally less than 1 gram of composition is required, in one embodiment from about 0.001 grams to about 0.5 grams and in another embodiment from about 0.1 gram to about 0.4 grams of composition is used. In one embodiment the amount of composition per square centimeter (cm) of material is less than about 1 gram/cm², in another embodiment less than about 0.2 grams/cm², in another embodiment from about 0.0001grams/cm² to about 0.1 grams/cm², and yet in another embodiment from about 0.01 grams/cm² to about 0.04 grams/cm².

The present invention may allow for a decreased frequency of application. For example, a 6% peroxide containing prior art composition that generally is used for 30 minutes twice daily for 2 weeks (e.g. for a total application time of 14 hours), may show substantially identical whitening efficacy by administering the same level of peroxide but used in accordance with the present invention, wherein the total application time is reduced to 6-10 hours. For example, when used in accordance with the present invention the same level of bleach agent may achieve equal or similar efficacy with one 30 minute application per day for 14 days or a 30 minute application twice daily for 7-10 days compared with a bleach composition of the prior art with the same bleach level.

Dental tray appliances may be used as follows. The patient or dental professional dispenses the present composition into a soft or rigid dental appliance and then the subject places the appliance over the subject's dental arch (or fits the device around his or her teeth to keep the tray in position). Generally, the recommended treatment period is in one embodiment a sufficient period of time to achieve whitening as disclosed above. At the end of the treatment period, the dental appliance is removed, cleaned with water to remove any remaining composition, and then stored until the next application.

The above-described compositions and delivery systems may be combined in a kit which comprises: 1. composition and 2. instructions for use; or comprises: 1. composition, 2. instructions for use, and 3. an integral carrier.

The compositions of this invention are useful for both human and other animals (e.g. pets, zoo, or domestic animals) applications.

### Other Uses

The compositions of the present invention can also be used in any situation where a source of bleach is desired. Such as but not limiting to, patches for bleaching stains on fabrics or hard surfaces, disinfecting patches for fabrics, hard surfaces or skin, bleaching patches for decoloring hairs.

### EXAMPLE

The following non-limiting example further describes an emulsion composition and its preparation method within the scope of the present invention. Many variations are possible without departing from the scope of the invention.

### Preparation of the Oil Gel (Hydrophobic Phase)

0,76kg of Kraton G 1651 (Styrenic Block copolymer) 0,76kg of Kraton G 1702(Styrenic Block copolymer) and 3,6g of Irganox 1010 (Antioxidant).are introduced into a Mixer Battagion IP 50. at 115°C and mixed for 1hour at 2000 Rpm.
8.5kg of Kaydol Oil (Mineral Hydrocarbon oil; 45%Napthenic 55% Paraffinic) are then added and mixed at 2000 RPM for other 3 hours . About 10 kg of Oil gel are obtained.

### Preparation of the Aqueous Phase

1.5 kg of Hydrogen Peroxide water solution at 35% by weight (Ultra Cosmetic Grade Solvay Interox) and 75 g of a Poly(Oxyethylene-co-Oxypropylene) Nonionic Surfactant (Pluronic PE 84,Basf) are Mixed at 200 rpm by helical ribbon impeller for 15 min in a beaker.

### Emulsification Process

A hot melt supply unit composed of a heated hopper with 5L capacity and a heated gear pump with a flow output of 8cc per revolution (Dynamini N05, Dynatech) is connected by a heated pipe with the head feed port of a intermeshing co-rotating twin-screw extruder (Thermo Prism Tse 24HC, Thermo Electron Company) having screw diameter 24mm and screw length 960mm, and a length expressed in terms of the length-to-diameter ratio (L/D) (40:1). Along the length of the twin-screw extruder 10 sections where it is possible to control the temperature independently, are identified.

Along the length of the twin-screw extruder are also identified two further feeding ports necessary to feed the emulsifier at low HLB (Sorbitan Monooleate) and the aqueous phase. The feeding port of the emulsifier is placed in the middle of the extruder length and the feeding port of the aqueous phase is placed at 3/4 of the extruder length., before the exit of the extruder. Both the Sorbitan Monooleate and the aqueous phase are supplied by peristaltic pumps.

Oil phase flow rate is set at 7.4 kg/h. Twin extruder is set at 1000 rpm and temperature on the head feeding port is set at 80°C, on the emulsifier feeding port at 50°C and on the aqueous phase feeding port at 35°C. Sorbitan Monooleate feeding flow rate is set at 0,5 kg/h and aqueous phase feeding flow rate is set at at 2.1 kg/h

The oil phase conveyed at 180°C from the hot melt supply unit is introduced from the head of the twin extruder were the temperature is reduced at 80°C and the twin screws are rotating at 1000 rpm. Here the oil phase is conveyed by the twin screws versus the feed port of emulsifier feeding where the temperature is reduced to 50°C and the oil phase is blended with the emulsifier. The blend of oil phase and emulsifier is conveyed by the twin screws towards the feed port of the aqueous phase feeding, where the temperature is reduced at 50°C, and is emulsified with the aqueous phase.

The emulsion is thus obtained with flow rate of 10 kg/h.

## Claims

1. A cohesive emulsion useful for bleaching teeth comprising:
a. an aqueous phase in an amount from 1% to 45%, preferably from 3% to 35% by weight of the composition
b. a safe and effective amount of a bleaching agent;
c. an inert hydrophobic phase in an amount from 30% to 98%, preferably from 40% to 90% by weight of the composition
d. an emulsifying system in an amount from 1% to 10%, preferably from 3% to 7% by weight of the composition
said composition being **characterized in that** said emulsifying system comprises at least one surfactant having an HLB<10, preferably comprised between 3 and 7, and at least one surfactant with HLB>10, preferably comprised between 12 and 16.

2. An emulsion according to claim1 which is capable of adhering to teeth.

3. An emulsion according to any preceding claim wherein said inert hydrophobic phase comprises a polymeric component and a hydrophobic oil.

4. An emulsion according to claim 3 wherein said polymeric component is a styrenic block copolymer.

5. An emulsion according to claims 3 or 4 wherein said hydrophobic oil is mineral oil.

6. An emulsion according to any preceding claim wherein said bleaching agent is hydrogen peroxide.

7. A method of making an emulsion which comprises the steps of:
a. combining an oil gel with a surfactant having an HLB<10 to form a hydrophobic phase.
b. combining water, a bleaching agent and a surfactant having an HLB>10 to form an aqueous phase
c. combining said hydrophobic phase and said aqueous phase in a twin screw extruder to form the emulsion

8. A method of making an emulsion according to claim 8 wherein step a) is preferably performed at a temperature ranging from 50°C to 100°C, step c) is preferably performed at a temperature ranging from 10°C to 60°C, more preferably from 20°C to 40°C, most preferably from 30°C to 40°C.

9. A method of making an emulsion according to claim 7 or 8 wherein the emulsion obtained is a composition according to claim 1.

10. An oral care delivery system comprising:
a. an integral carrier
b. a safe and effective amount of an emulsion according to any of claims 1 to 6.
